# EUROPÄISCHE PATENTSCHRIFT

(11) **EP 1 335 957 B1**
(45) Veröffentlichungstag und Bekanntmachung des Hinweises auf die Patenterteilung: **04.03.2009**
(21) Anmeldenummer: 01982371.5
(22) Anmeldetag: 27.09.2001
(51) Int. Cl.: A61K 8/891, A61K 8/02, A61K 8/37, A61K 8/39, A61K 8/58, A61K 8/895, A61Q 1/08, A61Q 1/10

(54) **KOSMETIKSTIFT**
COSMETIC PENCIL
CRAYON COSMETIQUE

(30) Priorität: 29.09.2000 DE 20016826 U
(43) Veröffentlichungstag der Anmeldung: 20.08.2003
(73) Patentinhaber: Schwan-STABILO Cosmetics GmbH & Co. KG, 90562 Heroldsberg (DE)
(72) Erfinder: WINKLER, Wolfgang, 91207 Lauf (DE); ZARLING, Claudia, 90408 Nürnberg (DE); LEBOK, Simona, 90409 Nürnberg (DE)
(74) Vertreter: Leissler-Gerstl, Gabriele
(86) Internationale Anmeldenummer: PCT/EP2001/011215
(87) Internationale Veröffentlichungsnummer: WO 2002/026903

(56) Entgegenhaltungen:
- EP-A- 0 836 846
- EP-A- 0 850 643
- EP-A- 1 035 181
- EP-A- 1 080 712
- DE-A- 19 911 748

## Beschreibung

Die Erfindung betrifft eine kosmetische Zubereitung gemäß Anspruch 1, vorzugsweise in Form von Stiften. Insbesondere betrifft diese Erfindung Lippenstifte, Lippenpflegestifte, Abdeckstifte (Concealer), Wangenrouge (Blusher), Lidschattenstifte, Lippenkonturenstifte, Augenkonturenstifte, Augenbrauenstifte, Sonnenschutzstifte, Antiakne-Stifte und vergleichbare Produkte, deren Minen in bekannter Weise durch Extrusion einer geeigneten Masse hergestellt und mit einer Umhüllung aus Holz, einem Holzersatzstoff oder einem Kunststoffmaterial versehen wurden. Grundsätzlich ist es auch möglich, die Minenmasse in bekannter Weise heiß in eine geeignete Umhüllung aus einem spitzbaren Material einzubringen und sie darin erkalten zu lassen. Die geeigneten Verfahren zur Umhüllung ausgeformter Minenmassen sind grundsätzlich von der Herstellung von Blei- und Farbstiften her bekannter Stand der Technik, welcher bereits 1927 auf die Herstellung von extrudierten Kosmetikstiften übertragen wurde.

Grundsätzlich bekannt ist auch, die Minen - je nach gewünschtem Anwendungszweck - als Konturenstift zum Zeichnen von Linien oder als Stift für flächigen Auftrag - mit unterschiedlichen Durchmessern herzustellen und weiter zu verarbeiten.

Vom Verbraucher erwünscht sind Texturen mit guten Applikationseigenschaften. Kosmetische Stifte der eingangs genannten Art sollen sich sanft und gleichmäßig auftragen lassen, konturenscharf zeichnen und möglichst lange am Applikationsort verbleiben. Sie sollen also weder durch die Hautfeuchtigkeit, Transpiration oder Sebum abgelöst werden und sich möglichst auch nicht auf andere Medien, wie Glas oder Textilien, übertragen.

Bei extrudierten Minen ist es bekannter Stand der Technik, eine Mischung aus Triglyceriden, Wachsen und Pigmenten - der Pigmentgehalt liegt vorzugsweise in einer Größenordnung von 40-50 Gew.-% - zu Minen zu verarbeiten. So hergestellte Minen zeichnen sich zwar durch einen satten Farbauftrag aus, sind aber relativ hart und eignen sich deshalb bevorzugt für Augenbrauenstifte sowie Augen- und Lippenkonturenstifte. Für die Herstellung von Lidschattenstiften sind solche Kosmetikmassen wegen ihrer Härte und des meist stumpfen Auftrags denkbar ungeeignet. Eine Verringerung des Pigmentgehalts und eine weichere Einstellung der Massen führt meist zu Problemen hinsichtlich der Deckkraft und zu einer verringerten Temperaturstabilität. Getreu dem "Prinzip des kleinsten Zwanges" (Le Chatelier, 1888) kommt es bei kosmetischen Massen, die Triglyceride enthalten, durch den hohen Druck beim Extrudieren meist zu Phasenumwandlungen in Kristallmodifikationen mit geringeren Volumina, was nach dem Extrudieren oft zu gummiartig-weichen Massen führt, welche erst nach längerer Lagerung wieder zu festeren Massen werden - das bekannte "Nachhärten" der Minen, mit oft unerwünschten Ausblühungen auf der Minenoberfläche.

Ein anderes Verfahren zur Herstellung von Minen ist das Gießen von bei erhöhter Temperatur aufgeschmolzener Mischungen von Ölen, Fetten, Wachsen und Pigmenten in meist aus Metall bestehende Gießformen und Weiterverarbeitung der Gießlinge nach dem Erkalten oder das direkte Eingießen der aufgeschmolzenen Massen in geeignete spitzbare Hülsen oder in geeignete Drehmechaniken. Bei den Gießverfahren ist die Einsatzmenge an Pigmenten begrenzt, da Pigmentkonzentrationen über 25-30 Gew.-% meist die Viskosität der flüssigen Massen so sehr erhöhen, daß ein Gießen nicht mehr möglich ist. Grundsätzlich ist es auch möglich, die bei erhöhter Temperatur flüssigen Massen unter Druck in Formen oder geeignete Hülsen einzupressen - aufgrund des vorgenannten "Prinzips von Le Chatelier" treten hierbei ebenfalls Schrumpfungs-prozesse auf, welche meist zu den bekannten, tiefen Tromben am hinteren Ende des erkalteten Gießlings führen. Zur Erreichung einer niedrigen Viskosität im aufgeschmolzenen Zustand müssen diesen kosmetischen Massen zudem Öle zugesetzt werden, welche später nachteilig für die Haltbarkeit dieser Massen sind. Eine gewisse Abhilfe konnte dadurch erreicht werden, daß man diesen kosmetischen Massen flüchtige Bestandteile, vorzugsweise kurzkettige lineare Siliconöle, cyclische Silicone, geeignete Isoparaffine oder Mischungen daraus, zugesetzt hat, was die spätere Haltbarkeit der in dieser Weise hergestellten kosmetischen Massen deutlich verbessert hat. Nachteilig ist aber hierbei, daß derart hergestellte Kosmetikstifte in hermetisch schließende Drehmechaniken oder in geeignete Hülsen aus Kunststoff eingefüllt werden und später mit dicht schließenden Kappen verschlossen werden mußten, um das durch Verdunsten der flüchtigen Bestandteile bedingte Austrocknen der Massen zu verhindern. Ein Eingießen solcher Massen in innenseitig unbehandelte Holzhülsen ist deshalb nicht möglich, da zum einen die in das Holz eingeschlossene Luft sich bei Kontakt mit der heißen Masse ausdehnt und den noch flüssigen Massen eine schwammartig-poröse Struktur verleiht und da zum anderen die flüchtigen Bestandteile durch das Holz diffundieren und die eingegossenen Minen dann schrumpfen lassen. Verfahren, die Innenseiten von Holzhülsen abzudichten, sind hinlänglich bekannt - dennoch konnten sich die so hergestellten Kosmetikstifte im Markt nicht durchsetzen.

Zur Herstellung von Kosmetikmassen, die auf die Haut aufgetragen werden sollen und dort haften sollen, wird vielfach vorgeschlagen, Siliconverbindungen zu verwenden. So beschreibt A. Domsch in seinem Buch "Die kosmetischen Präparate", Bd. III, Lipidhaltige und emulgierte Formulierungen, 4. Auflage (1994), Verlag für chemische Industrie, Augsburg, Zusammensetzungen für Make-up-Stifte, die aus Wachs, flüchtigen Siliconen, Pigment und Fettsäureethern und -estern aufgebaut sein können. Der Pigmentanteil in diesen Stiften liegt allerdings nur bei 10 bis 20%.

In JP-A 07 233 025 wird vorgeschlagen, zur Verbesserung der Haftfähigkeit einer kosmetischen Zusammensetzung auf der Haut, ein Polyether-modifiziertes Silicon zuzusetzen.

Der Zusatz von siliconhaltigen Verbindungen ist auch gemäß EP-A 0 850 643 und in EP-A 0 850 644 für die Herstellung kosmetischer Zubereitungen, insbesondere für weiche Pasten, vorgesehen. Auch der Einsatz der verschiedensten Ester in Form von Ölen, Fetten oder auch synthetischer Ester wird dort beschrieben. So werden beispielsweise kosmetische Zubereitungen beschrieben, die esterhaltige Öle enthalten. Allerdings bilden die gemäß diesen beiden Literaturstellen erhaltenen Massen bei der Extrusion weiche Pasten, die somit nicht für die Herstellung von Kosmetikstiften geeignet sind.

Aus JP-A 2 229 106 sind feste kosmetische Massen bekannt, die zur Verbesserung der Eigenschaften ein mehrwertiges Metallsalz eines Dialkylphosphats neben einem oder mehreren lipophilen Estern, Siliconöl, einem kosmetischen Öl sowie Pigment enthalten. Das damit herzustellende Make-up zeichnet sich dadurch aus, dass es leicht aufgetragen werden kann und eine hohe Deckkraft besitzt.

Weiterhin sind aus EP-A 0 660 701 und EP-A 0 685 226 kosmetische Sonnenschutzmittel bekannt, die spezielle UV-Licht filternde Verbindungen enthalten und darüber hinaus ein Öl enthalten, das aus mehrwertigen Estern ausgewählt ist. Diese Zusammensetzungen werden in Form von Lotionen, Gelen, Emulsionen, Cremes, Milch und dgl. formuliert.

Aufgabe der Erfindung war es nun, ausgehend von diesen bekannten Zusammensetzungen eine kosmetische Masse zu entwickeln, welche sich zur Herstellung extrudierter Minen eignet, die, insbesondere für Lidschattenstifte und Blusher, einen flächigen Auftrag ermöglicht, und die andererseits auch für Konturenstifte verwendet werden kann - kosmetische Stifte also, welche die gleichen Gebrauchseigenschaften zeigen, wie die durch Gießen von geeigneten Massen bei erhöhter Temperatur zu erhaltenden Produkte. Solcherart hergestellte Minen unterschiedlicher Durchmesser sollen nach dem Extrudieren vorzugsweise in Holz oder in Holzersatzstoffe eingeleimt und zu fertigen Stiften weiterverarbeitet werden. Sie sollen sich durch ein cremig-weiches Applikationverhalten, verbesserte Haltbarkeit und verringerten Transfer auf andere Medien, gute Lagerfähigkeit, ohne Veränderung der Gebrauchseigenschaften, und gute Temperaturstabilität auszeichnen. Zudem sollte bei der Entwicklung solcher kosmetischer Massen auf die Verwendung flüchtiger Bestandteile verzichtet werden.

Die Aufgabe der Erfindung wird mit der in Anspruch 1 definierten kosmetischen Zusammensetzung sowie mit der in dem Anspruch 9 definierten Verwendung gelöst.

Überraschenderweise wurde festgestellt, dass durch die Kombination des speziell ausgewählten Esters mit dem speziellen Siliconwachs und durch Verzicht auf die Verwendung von Triglyceriden mit einem Schmelzbereich zwischen 30 und 60°C Massen hergestellt werden können, die durch Extrusion zu Minen geformt werden können, die ausgezeichnete kosmetische und mechanische Eigenschaften besitzen.

Die Aufgabe der Erfindung wurde somit gelöst, indem auf den Einsatz von festen Triglyceriden, insbesondere von Triglyceriden mit einem Schmelzbereich zwischen 30-60 °C verzichtet wurde. Diese wurden ersetzt durch spezifische Ester von geradkettigen oder verzweigten Carbonsäuren mit geradkettigen oder verzweigten Alkoholen mit einer Kettenlänge zwischen C₁ und C₂₄, bevorzugt durch Ester der Zitronensäure mit mittleren Kettenlängen der Alkylketten, bspw. Tri-(Cetyl-/Stearyl)-citrat und/oder Methylglucose-distearat und/oder Palmitoyl-/Stearoyl-monoglycerid oder deren Gemische. Als geeignet hat sich auch Isostearylisostearat erwiesen. Besonders gute Ergebnisse wurden erzielt, wenn man die genannten Ester mit Siliconwachsen, wie bspw. Stearyl Dimethicone und polymeren Siloxysilicaten der allgemeinen Formel [(CH₃)₂SiO_{½}]ₓ [SiO₂]_{y} oder Siliconelastomeren vom Typ DimethiconeNinyldimethicone Crosspolymer oder Mischungen der genannten Silicon-Derivate kombinierte. Für die vorliegende Erfindung geeignete polymere Siloxysilicate werden beispielsweise in "International Cosmetic Ingredient Dictionary and Handbook", 7. Ausgabe, 1997, Band 2, beschrieben und sind im Handel erhältlich. Die Siloxysilicate entsprechen der oben Die Siloxysilicate entsprechen der oben angegebenen Formel, wobei bevorzugt solche Siloxysilicate ausgewählt werden, bei denen in der Formel x und y solche Werte annehmen, dass jeweils die gewünschten Eigenschaften erzielt werden, wie es dem Fachmann bekannt ist. Das Verhältnis der Esterkomponenten zu Siliconkomponenten liegt dabei in einem Bereich zwischen 1 : 5 bis 2:1. Bevorzugt wird ein Bereich zwischen 1 : 4 und 1:1.

In einer bevorzugten Ausführungsform wird als Ester ein Ester der Zitronensäure mit Cetyl- und/oder Stearoylalkohol, ein Monoglycerid von Palmitin- und/oder Stearinsäure oder ein Ester, der sich von Methylglucose und Stearinsäure ableitet, verwendet. In einer anderen bevorzugten Ausführungsform wird als Ester ein Saccharoseester, insbesondere ein Saccharoseester von Fettsäuren mit mittlerer Kettenlänge, wie Sucroselaurat, Sucrosemyristat, Sucrosepalmitat, Sucrosestearat, Sucrose-tetrastearat-triacetat oder eine Mischung davon, vorzugsweise das höherschmelzende Sucrose-tetrastearat-triacetat, gegebenenfalls gemischt mit einem anderen der oben beschriebenen Ester verwendet.

Es zeigte sich, daß zur Herstellung der erwünschten kosmetischen Massen mit cremigem Auftrag und langer Haltbarkeit auf der Haut die vorgenannten Ester und Silicon-Derivate problemlos mit üblichen Ölkomponenten, wie bspw. Caprylic/Capric Triglycerides, Octyldodecanol, Butylstearat und mit üblichen Wachskomponenten, wie bspw. Bienenwachs, Carnaubawachs, Candelillawachs, Ouricuriwachs, Apfelwachs, Japanwachs, mikrokristallines Wachs, Ozokerit, synthetischem Wachs, hydriertem Ricinusöl u.a. zur Erreichung des gewünschten Zwecks kombiniert werden können.

Überraschend wurde gefunden, daß in diese Grundmassen mit einem cremigen Hautgefühl auch höhere Mengen an Pigmenten - bis etwa 50 Gew.-% und in Kombination mit Perlglanzpigmenten auf Basis von Glimmern auch noch darüber - möglich werden, ohne die erwünschten Produkteigenschaften nachteilig zu verändern.

Als Pigmente geeignet und für Kosmetika einsetzbar sind grundsätzlich die in der Kosmetik-Verordnung im Anhang 3 (zu § 3) genannten Färbemittel oder solche, die durch die nationalen Gesetzgebungen in den U.S.A. oder in Japan zugelassen sind. Beispielhaft, ohne die Verwendung einzuschränken, seien hier genannt: Titandioxid, Eisenoxide, Ultramarin, Chromoxidgrün, Chromoxidhydratgrün, Berliner Blau, Zinkoxid, Glimmer, mit Metalloxiden beschichtete Glimmer, Bismut-oxychlorid, Metallpulver, wie z.B. plättchenförmiges Aluminium, Kupfer, Bronze, Messing, Silber oder Gold, Carmin, organische Pigmente, unlösliche Verlackungen organischer Farbstoffe oder Mischungen daraus. Daneben können weitere Feststoffe als Füllstoffe und Konsistenzregler eingesetzt werden, wie z.B. Talkum, Kaolin, Bentonit, Hectorit, Montmorillonit, Smectit, Magnesium-Aluminium-Silicat oder in Wasser unlösliche Metallseifen, wie z.B. Aluminium-, Magnesium-, Calcium- oder Zinkstearat oder Mischungen der genannten Feststoffe.

Die Herstellung der kosmetischen Massen erfolgt, indem man die lipophilen Komponenten zusammengibt und bei erhöhter Temperatur - etwa 10 °C oberhalb der Schmelztemperatur der höchstschmelzenden Komponente - klar durchschmelzen läßt und ggf. filtriert. Danach werden alle Feststoffe wie Pigmente, Füllstoffe, Antiabsetzmittel usw. und ggf. Konservierungsmittel, Antioxidantien, Riechstoffe, Wirkstoffe und sonstige Zusätze zugegeben und das Ganze homogenisiert. Möglich ist auch, den Massen zur Verbesserung der kosmetischen Eigenschaften hydrophile Komponenten, wie bspw. Wasser, Glycerin, 1.2-Propylenglykol, 1.2-Hexylenglykol, Pantothenol und auch Tocopherylacetat oder Tocopherylinoleat oder Mischungen der genannten Substanzen, vor dem Homogenisieren zuzusetzen.

Anschließend wird zum Erreichen einer einheitlichen Teilchengröße mit einer Korundscheibenmühle oder einer Perlenmühle oder einem Dreiwalzenstuhl vermahlen. Danach wird die Masse entlüftet und mit einem geeigneten Extruder zu Minen verpreßt. Die Minen werden anschließend in üblicher Weise in Holzbrettchen eingeleimt und zu Kosmetikstiften verarbeitet.

Es wurde erfindungsgemäß festgestellt, dass gerade die Kombination aus einem Ester, wie er in den Ansprüchen definiert ist, und einem Siliconwachs dazu führt, dass eine kosmetische Masse, die üblicherweise aus einer lipophilen Phase mit Wachs und Öl besteht, extrudiertähig wird und bei Extrusion solche mechanischen Eigenschaften erhält, dass sie problemlos zu Minen und dann zu Stiften verarbeitet werden kann.

Ein weiterer Gegenstand der Erfindung ist daher die Verwendung einer Kombination aus a) einem Ester einer geradkettigen oder verzweigten C₁-C₂₄-Carbonsäure und einem geradkettigen oder verzweigten C₁-C₂₄-Alkohol und b) einem Siliconwachs als Zusatz zu einer kosmetischen Zubereitung, die mindestens eine lipophile Phase aufweist und neben Ölen und Wachsen die üblichen Inhaltstoffe enthält, um die kosmetische Zubereitung durch Extrusion formbar zu machen. Gegenstand der Erfindung ist auch die Verwendung der genannten Kombination zur Herstellung einer extrudierbaren kosmetischen Zubereitung.

Nachstehend soll die Erfindung anhand einiger Beispiele erläutert werden ohne sie dadurch einzuengen. Die Mengenangaben erfolgen in Gewichts-Prozent (Gew.-%), die Rohstoffe werden mit den international üblichen INCI-Namen bezeichnet.

### Beispiel 1

| | |
|---|---|
| Caprylic/Capric Triglycerides | 8,000 |
| Polyglyceryl-3 Methylglucose Distearate | 2,000 |
| Tri C 14-15 Alkyl Citrate | 5,000 |
| Hydrogenated Palm Glycerides-Monoglyceride | 2,500 |
| Synthetic Wax | 1,000 |
| Stearyl Dimethicone | 20,000 |
| Trimethylsiloxysilicate | 7,500 |
| Dimethicone/Vinyldimethicone Crosspolymer | 2,500 |
| Pigments, Mica, Kaolin | 51,000 |
| Antioxidants | 0,500 |
| | 100,000 |

Als Färbemittel werden anorganische Pigmente und mit Titandioxid und ggf. weiteren Metalloxiden beschichtete Glimmer verwendet. Die Fettbestandteile werden bei 100 °C aufgeschmolzen, danach werden die Pigmente, Füllstoffe und Siliconpolymere zugesetzt und die Masse mit einem Dreiwalzenstuhl gewalzt und anschließend zu Minen für einen Lidschattenstift extrudiert.

### Beispiel 2

| | |
|---|---|
| Capric/Caprylic Triglycerides | 2,500 |
| Polyglyceryl-3 Methylglucose Distearate | 1,500 |
| Tri C14-15 Alkyl Citrate | 5,000 |
| Hydrogenated Palm Glycerides-Monoglycerides | 3,000 |
| Synthetic Wax | 1,500 |
| Stearyl Dimethicone | 19,500 |
| Trimethylsiloxysilicate | 8,000 |
| Pigments, Kaolin, Mica | 52,500 |
| Aqua | 5,000 |
| Preservatives, Antioxidants | 1,500 |
| | 100,000 |

Das Wasser (destilliertes Wasser) wird auf 70 °C erwärmt und das Konservierungsmittel-System darin gelöst. Die Fettbestandteile werden bei 100 °C aufgeschmolzen und auf 80 °C abkühlen lassen. Die Wasserphase wird zugegeben und der Ansatz intensiv gemischt. Danach werden die Pigmente, Füllstoffe und die Siliconpolymere zugesetzt. Anschließend wird die Mischung in einem Kneter 30 min geknetet. Anschließend wird die Masse auf einem Dreiwalzenstuhl gewalzt und extrudiert. Vor dem Einleimen werden die Minen bei 50 °C im Vakuum (40-50 kPa) getrocknet. Bei Verwendung anorganischer Pigmente und mit Titandioxid und ggf. mit weiteren Metalloxiden beschichteten Glimmern lassen sich Minen für Lidschattenstifte herstellen. Bei Verwendung von Eisenoxiden in Abmischung mit Titandioxid werden Minen für Korrekturstifte und Blusher erhalten.

### Beispiel 3

| | |
|---|---|
| Bienenwachs | 3,500 |
| Ouricuriwachs | 2,500 |
| Isostearyl-isostearat | 5,000 |
| Polyglyceryl-3 Methylglucose Distearate | 1,000 |
| Hydrogenated Palm Glycerides-Monoglycerides | 3,000 |
| Stearyl Dimethicone | 10,000 |
| Sucrose Tetrastearate Triacetate | 8,000 |
| Sucrose Laurate | 1,000 |
| Trimethylsiloxysilicate | 7,500 |
| Pigments, Kaolin, Mica | 49,000 |
| Aqua | 3,500 |
| D-Pantothenol | 2,500 |
| 1.2-Hexylenglykol | 2,000 |
| Preservatives, Antioxidants | 1,500 |
| | 100,000 |

Die Herstellung erfolgt analog zu Beispiel 2. Die Masse eignet sich für Lippenstifte mit hautpflegenden Eigenschaften. Ersetzt man etwa 15-20 Gew.-% der angegebenen Pigmentmenge durch feinteiliges Titandioxid mit Teilchengrößen um 20-50 nm, so lassen sich Lippenstifte mit hohem Lichtschutzfaktor oder - bei Auswahl geeigneter anorganischer Pigmente oder organischer Pigmente oder Farblacke oder Gemischen daraus - Stifte zur Körperbemalung mit hohem Lichtschutzfaktor herstellen, wie sie bspw. bei Surfern oder Skifahrern beliebt sind. Möglich ist auch, durch Auswahl geeigneter Pigmente, Stifte in hautfarbenen Tönungen herzustellen, welche sich für Zwecke der Camouflage, zum Abdecken von Hautanomalien, eignen.

## Patentansprüche

1. Kosmetische Zubereitung in Form einer Mine enthaltend eine lipophile Phase, bestehend aus bei 25°C flüssigen Ölen, Wachsen und einer Feststoffphase, wobei die lipophile Phase eine Kombination aus
a) einem Ester ausgewählt aus Estern der Citronensäure mit Cetylalkohol, Estern der Citronensäure mit Stearylalkohol, Monoglyceriden der Palmitin- oder Stearinsäure oder Estern, die sich von Methylglucose und Stearinsäure ableiten, Saccharoseestern mit C₁₂-C₂₀-Fettsäuren, Saccharosetetrastearattriacetat, Isostearylisostearat und/oder Polyglycerin-3-methylglucosedistearat oder deren Mischungen und
b) einem Siliconwachs ausgewählt aus Stearyldimethicone, polymeren Siloxysilicaten der allgemeinen Formel [(CH₃)₂SiO_{1/2}]ₓ[SiO₂]_{y} oder Siliconelastomeren vom Typ DimethiconeNinyldimethicone-Crosspolymer oder Mischungen davon enthält,
wobei die Zubereitung frei ist von festen Triglyceriden mit einem Schmelzbereich zwischen 30 und 60°C und wobei das Verhältnis der Esterkomponente zur Silikonkomponente in einem Bereich zwischen 1 : 5 bis 2 : 1 liegt.

2. Kosmetische Zubereitung nach Anspruch 1, **dadurch gekennzeichnet, dass** die lipophile Phase als Komponente (a) Tri-(Cetyl/Stearyl)-Citrat und/oder Methylglucosedistearat und/oder Palmitoyl/Stearoyl-Monoglycerid und/oder Isostearylisostearat enthält.

3. Kosmetische Zubereitung nach einem der vorhergehenden Ansprüche, **dadurch gekennzeichnet, dass** sie als Komponente a) Ester der Zitronensäure mit Cetyl- und Stearylalkohol, Monoglyceride der Palmitin- und Stearinsäure oder Ester, die sich von der Methylglucose und Stearinsäure ableiten oder deren Mischungen enthält.

4. Kosmetische Zubereitung nach einem der vorhergehenden Ansprüche, **dadurch gekennzeichnet, dass** sie Sucrose Laurate, Sucrose Myristate, Sucrose Palmitate, Sucrose Stearate, Sucrose Tetrastearate Triacetate oder deren Gemische enthält.

5. Kosmetische Zubereitung nach einem der vorhergehenden Ansprüche, **dadurch gekennzeichnet, dass** sie als Feststoffphase für Kosmetika zugelassene Färbemittel, Perlglanzpigmente auf Basis von Glimmer, plättchenförmige Metallpulver, Talkum, Kaolin, Bentonit, Hectorit, Montmorillonit, Smectit, Magnesium-Aluminium-Silicat, in Wasser unlösliche Metallseifen oder deren Gemische enthält.

6. Kosmetische Zubereitung nach einem der vorhergehenden Ansprüche, **dadurch gekennzeichnet, dass** sie Aluminiumstearat, Magnesiumstearat, Calciumstearat, Zinkstearat oder Mischungen daraus enthält.

7. Kosmetische Zubereitung nach einem der vorhergehenden Ansprüche, **dadurch gekennzeichnet, dass** sie bis etwa 50 Gew.-% Pigmente enthält.

8. Kosmetische Zubereitung nach einem der vorhergehenden Ansprüche, **dadurch gekennzeichnet, dass** die Mine durch Extrusion der kosmetischen Zubereitung erhalten wurde.

9. Verwendung einer Kombination aus
a) einem Ester ausgewählt aus Estern der Citronensäure mit Cetylalkohol, Estern der Citronensäure mit Stearylalkohol, Monoglyceriden der Palmitin-oder Stearinsäure oder Estern, die sich von Methylglucose und Stearinsäure ableiten, Saccharoseestern mit C₁₂-C₂₀-Fettsäuren, Saccharosetetrastearattriacetat, Polyglycerin-3-methylglucosedistearat und/oder Isostearylisostearat oder deren Mischungen und
b) einem Siliconwachs ausgewählt aus Stearyldimethicone, polymeren Siloxysilicaten der allgemeinen Formel [(CH₃)₂SiO_{1/2}]ₓ[SiO₂]_{y} oder Siliconelastomeren vom Typ Dimethicone/Vinyldimethicone-Crosspolymer oder Mischungen davon,
zur Herstellung einer zu Minen extrudierbaren kosmetischen Zubereitung, die frei ist von festen Triglyceriden mit einem Schmelzbereich zwischen 30 und 60°C.

## Claims

1. Cosmetic preparation in the form of a filler element containing a lipophilic phase, consisting of oils that are liquid at 25°C, waxes and a solid phase, wherein the lipophilic phase contains a combination of
a) an ester selected from esters of citric acid with cetyl alcohol, esters of citric acid with stearyl alcohol, monoglycerides of palmitic or stearic acid or esters that are derived from methylglucose and stearic acid, saccharose esters with C₁₂-C₂₀ fatty acids, saccharose tetrastearate triacetate, isostearyl isostearate and/or polyglycerol-3-methylglucose distearate or mixtures thereof, and
b) a silicone wax selected from stearyl dimethicone, polymer siloxysilicates of the general formula [(CH₃)₂SiO_{1/2}]ₓ[SiO₂]_{y} or silicone elastomers of the dimethicone/vinyl-dimethicone-crosspolymer type or mixtures thereof,
wherein the preparation is free of solid triglycerides with a melting range between 30 and 60°C, and wherein the ratio of the ester component to the silicone component lies in a range between 1 : 5 and 2 : 1.

2. Cosmetic preparation according to claim 1, **characterised in that** the lipophilic phase contains as component (a) tri-(cetyl/stearyl)-citrate and/or methylglucose distearate and/or palmitoyl/stearoyl monoglyceride and/or isostearyl isostearate.

3. Cosmetic preparation according to one of the preceding claims, **characterised in that** it contains as component a) esters of citric acid with cetyl and stearyl alcohol, monoglycerides of palmitic and stearic acid or esters that are derived from methylglucose and stearic acid or mixtures thereof.

4. Cosmetic preparation according to one of the preceding claims, **characterised in that** it contains sucrose laurate, sucrose myristate, sucrose palmitate, sucrose stearate, sucrose tetrastearate triacetate or mixes thereof.

5. Cosmetic preparation according to one of the preceding claims, **characterised in that** it contains as the solid phase colouring agents permitted for cosmetics, mica-based pearly lustre pigments, platelike metal powders, talcum, kaolin, bentonite, hectorite, montmorillonite, smectite, magnesium-aluminium silicate, metallic soaps that are insoluble in water or mixes thereof.

6. Cosmetic preparation according to one of the preceding claims, **characterised in that** it contains aluminium stearate, magnesium stearate, calcium stearate, zinc stearate or mixtures thereof.

7. Cosmetic preparation according to one of the preceding claims, **characterised in that** it contains up to approximately 50 % by weight pigments.

8. Cosmetic preparation according to one of the preceding claims, **characterised in that** the filler element has been obtained by extrusion of the cosmetic preparation.

9. Use of a combination consisting of
a) an ester selected from esters of citric acid with cetyl alcohol, esters of citric acid with stearyl alcohol, monoglycerides of palmitic or stearic acid or esters that are derived from methylglucose and stearic acid, saccharose esters with C₁₂-C₂₀ fatty acids, saccharose tetrastearate triacetate, polyglycerol-3-methylglucose distearate and/or isostearyl isostearate or mixtures thereof, and
b) a silicone wax selected from stearyl dimethicone, polymer siloxysilicates of the general formula [(CH₃)₂SiO_{1/2}]ₓ[SiO₂]_{y} or silicone elastomers of the dimethicone/vinyl-dimethicone-crosspolymer type or mixtures thereof,
for the production of a cosmetic preparation that can be extruded to form filler elements and is free of solid triglycerides with a melting range between 30 and 60°C.

## Revendications

1. Composition cosmétique sous la forme d'une mine, contenant une phase lipophile, composée d'huiles liquides à 25 °C, de cires et d'une phase solide, la phase lipophile contenant une combinaison
a) d'un ester sélectionné parmi des esters de l'acide citrique avec un alcool de cétyle, des esters de l'acide citrique avec un alcool de stéaryle, des monoglycérides de l'acide palmitique ou stéarique ou des esters qui dérivent du méthylglucose et de l'acide stéarique, des esters de saccharose avec des acides gras en C₁₂-C₂₀, du tétrastéarate-triacétate de saccharose, de l'isostéarate d'isostéaryle et/ou du distéarate de polyglycérine-3-méthylglucose ou leurs mélanges, et
b) d'une cire de silicone sélectionnée parmi la stéaryldiméthicone, des siloxysilicates polymères de formule générale [(CH₃)₂SiO_{1/2}]ₓ[SiO₂]_{y} ou des élastomères de silicone du type diméthicone/vinyldiméthicone-polymère réticulé ou leurs mélanges,
dans laquelle la composition est exempte de triglycérides solides ayant une plage de fusion comprise entre 30 et 60 °C et dans laquelle le rapport entre composante ester et composante silicone est compris dans une gamme allant de 1:5 à 2:1.

2. Composition cosmétique selon la revendication 1, **caractérisée en ce que** la phase lipophile contient en tant que composante (a) du tri-citrate de (cétyle/stéaryle) et/ou du distéarate de méthylglucose et/ou du monoglycéride de palmitoyle/stéaroyle et/ou de l'isostéarate d'isostéaryle.

3. Composition cosmétique selon l'une des revendications précédentes, **caractérisée en ce qu'**elle contient en tant que composante a) des esters de l'acide citrique avec de l'alcool de cétyle et de stéaryle, des monoglycérides de l'acide palmitique et stéarique ou des esters qui dérivent du méthylglucose et de l'acide stéarique ou leurs mélanges.

4. Composition cosmétique selon l'une des revendications précédentes, **caractérisée en ce qu'**elle contient du laurate de saccharose, du myristate de saccharose, du palmitate de saccharose, du stéarate de saccharose, du tétrastéarate-triacétate de saccharose ou leurs mélanges.

5. Composition cosmétique selon l'une des revendications précédentes, **caractérisée en ce qu'**elle contient en tant que phase solide des colorants autorisés en cosmétique, des pigments lustrants à base de mica, de la poudre métallique en forme de paillettes, du talc, du kaolin, de la bentonite, de l'hectorite, de la montmorillonite, de la smectite, du silicate de magnésium-aluminium, des savons métalliques insolubles dans l'eau ou leurs mélanges.

6. Composition cosmétique selon l'une des revendications précédentes, **caractérisée en ce qu'**elle contient du stéarate d'aluminium, du stéarate de magnésium, du stéarate de calcium, du stéarate de zinc ou leurs mélanges.

7. Composition cosmétique selon l'une des revendications précédentes, **caractérisée en ce qu'**elle contient jusqu'environ 50 % en masse de pigments.

8. Composition cosmétique selon l'une des revendications précédentes, **caractérisée en ce que** la mine a été obtenue par extrusion de la composition cosmétique.

9. Utilisation d'une combinaison
a) d'un ester sélectionné parmi des esters de l'acide citrique avec un alcool de cétyle, des esters de l'acide citrique avec un alcool de stéaryle, des monoglycérides de l'acide palmitique ou stéarique ou des esters qui dérivent du méthylglucose et de l'acide stéarique, des esters de saccharose avec des acides gras en C₁₂-C₂₀, du tétrastéarate-triacétate de saccharose, du distéarate de polyglycérine-3-méthylglucose et/ou de l'isostéarate d'isostéaryle ou leurs mélanges, et
b) d'une cire de silicone sélectionnée parmi la stéaryldiméthicone, des siloxysilicates polymères de formule générale [(CH₃)₂SiO_{1/2}]ₓ[SiO₂]_{y} ou des élastomères de silicone du type diméthicone/vinyldiméthicone-polymère réticulé ou leurs mélanges,
pour fabriquer une composition cosmétique pouvant être extrudée en une mine, qui est exempte de triglycérides solides ayant une plage de fusion comprise entre 30 et 60 °C.
